# FASCICULE DE BREVET EUROPEEN

(11) **EP 0 447 909 B1**
(45) Date de publication et mention de la délivrance du brevet: **20.07.1994**
(21) Numéro de dépôt: 91103664.8
(22) Date de dépôt: 11.03.1991
(51) Int. Cl.: A61M 5/142, A61M 5/50, F04B 43/12

(54) **Pompe portable d'administration d'une substance thérapeutique liquide**
Tragbare Pumpe zur Verabreichung therapeutischer Flüssigkeiten
Portable pump for the administration of therapeutic liquids

(30) Priorité: 23.03.1990 FR 9003869
(43) Date de publication de la demande: 25.09.1991
(73) Titulaire: ASULAB S.A., CH-2502 Bienne (CH)
(72) Inventeur: Aubert, Christophe, CH-2052 Fontainemelon (CH); Dubois, Antoine, F-25130 Villers-le-Lac (CH)
(74) Mandataire: Caron, Gérard

(56) Documents cités:
- EP-A- 0 275 213
- EP-A- 0 275 214
- FR-A- 2 632 529
- GB-A- 2 120 003

## Description

La présente invention concerne une pompe portable à réservoir intégré, permettant l'administration d'une substance thérapeutique liquide.

Les pompes médicales sont connues depuis au moins une dizaine d'années et permettent d'administrer à un patient des médicaments sous forme liquide. Ce type de pompe portable trouve une application particulière, par exemple, pour le traitement des diabétiques, des cancéreux et des malades atteints du sida, qui ont besoin de recevoir des médicaments à petites doses et en continu, sans pour autant être obligatoirement alités et reliés à un appareillage médical encombrant. Ces pompes de petites dimensions prévues pour avoir une autonomie de plusieurs jours, permettent au patient de circuler librement. Il est seulement nécessaire de remplir le réservoir régulièrement.

On utilise fréquemment dans le domaine médical des pompes péristaltiques.

La demande de brevet internationale W0 82/04291 décrit une pompe péristaltique dont le principe est bien connu. Il consiste à utiliser un tube en matière plastique déformable, à l'écraser localement contre un carter au moyen d'un rotor équipé de galets presseurs, le rotor étant entraîné en rotation par un moteur, pour aspirer le liquide contenu dans le tube et provenant d'un réservoir, puis le refouler vers la sortie de la pompe.

La demande de brevet français FR 8904044 au nom de la Demanderesse décrit également un tel type de pompe, appliqué au domaine médical. Cette pompe comprend une "unité pompe" proprement dite, reliée par un tuyau flexible à un réservoir indépendant constitué par une vessie en matière plastique souple. Cette unité pompe présente deux modules, un module moteur non stérilisable comprenant un moteur et son circuit de commande et un module pompe, stérilisable, comprenant un rotor muni de ses galets, un tuyau et un presseur coopérant avec les galets pour l'écrasement dudit tuyau. Ces deux modules sont assemblables de façon amovible.

Cette pompe en deux parties amovibles, déjà connue, peut être facilement stérilisée, car elle est réalisée de façon que les organes ne résistant pas à la stérilisation, soient exclusivement logés dans l'une des parties. Cependant, cette pompe peut être utilisée plusieurs fois et pour des buts divers par un utilisateur quelconque, par exemple un malade ayant reçu cette pompe de son médecin traitant. Ceci peut poser des problèmes d'utilisation non-adéquate de la pompe engendrant par exemple une perte de stérilité du médicament administré ou d'utilisation détournée de la pompe.

Il est également connu du document EP 275 213 un dispositif d'administration d'un médicament en continu à l'aide d'une pompe.

Dans ce document, il est prévu un dispositif comportant une première unité, comprenant des moyens de pompage et une batterie, et une deuxième unité comprenant un moteur, destiné a actionner les moyens de pompage, et une électronique de commande.

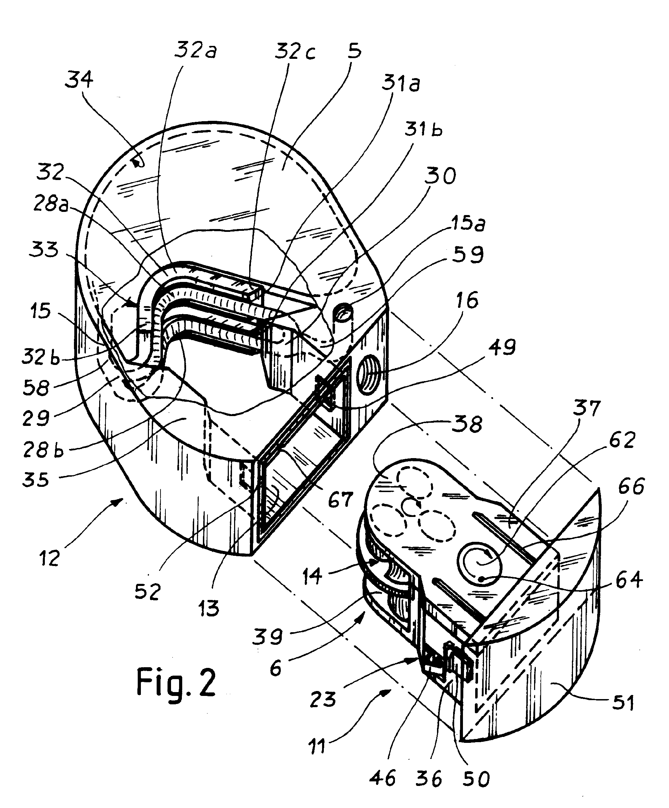

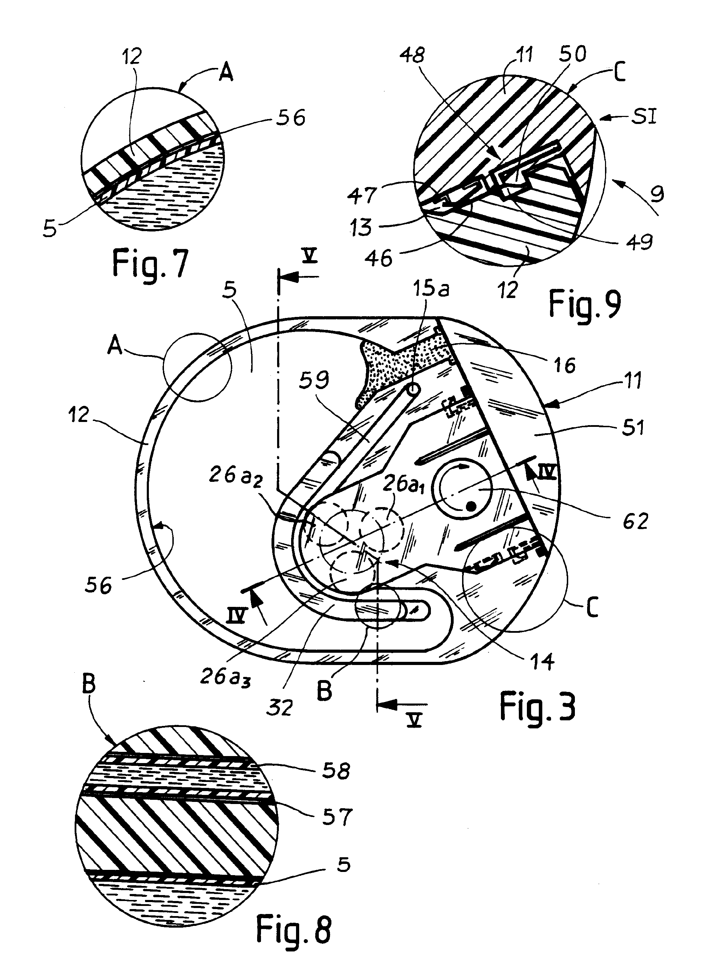

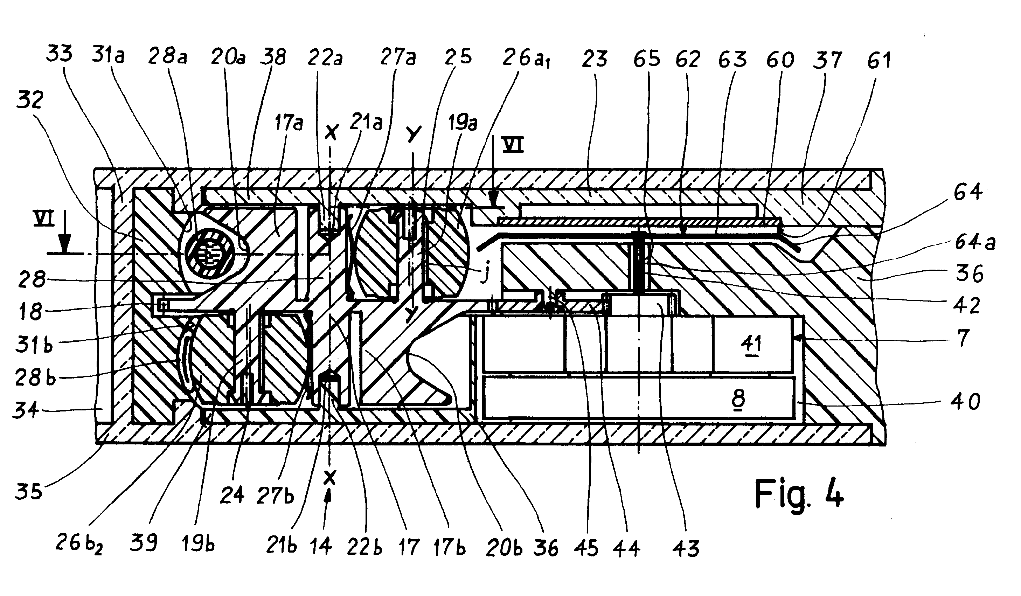

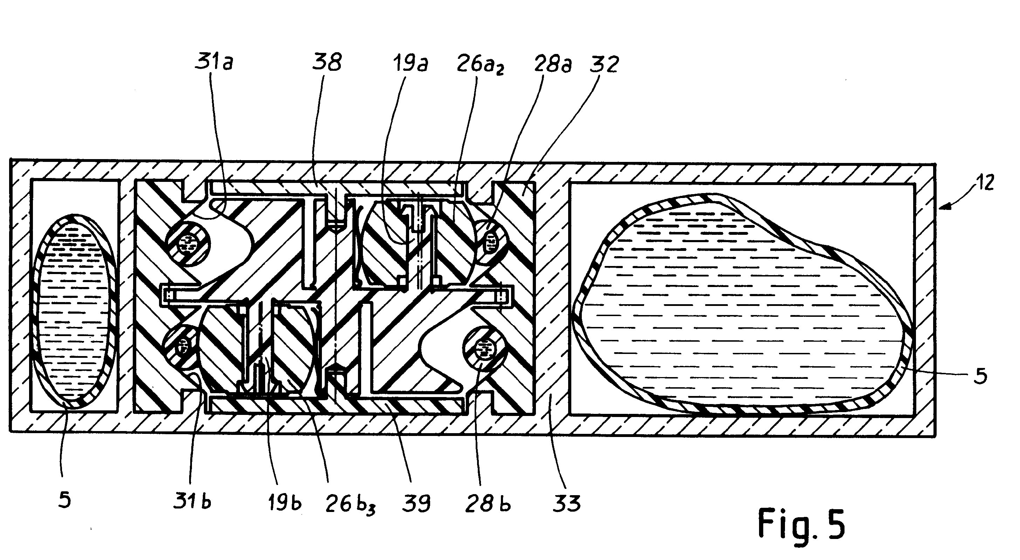

Selon ce document, la deuxième unité sert à plusieurs administrations médicamenteuses et la permière unité est jetable après un seul emploi. Afin qu'un utilisateur n'emploie qu'une seule fois la première unité, il est prévu trois variantes de réalisation suivantes :
- une variante (figure 2) avec un petit mécanisme empêchant l'assemblage des première et deuxième unités lorsque ces deux unités ont déjà été une fois assemblées, ceci étant réalisé en brisant un téton appartenant à la première unité lors d'un premier assemblage;
- une variante (figure 3) avec un système de décharge de la batterie lorsque les première et deuxième unités sont assemblées pour une seconde fois, ceci étant réalisé par le brisement d'une languette d'isolation;
- une variante (figure 4) dans laquelle une languette conductrice, appartenant à la première unité, est brisée lors d'un premier assemblage et appliquée à l'aide d'un ressort contre deux bornes externes du circuit d'alimentation du moteur de manière a fermer le circuit d'alimentation, la languette conductrice étant séparée de la première unité lors d'un désassemblage suivant un premier assemblage des première et deuxième unités.

On remarquera premièrement que le dispositif décrit ci-avant est conçu de manière à indiquer, par prévention, à un utilisateur si l'unité jetable à déjà été utilisée. Cet utilisateur s'en apercevra par le fait qu'il ne peut pas assembler les première et deuxième unités mentionnées ou par le fait que le dispositif ne fonctionne pas lorsqu'il veut enclencher la pompe à l'aide d'un interrupteur situé sur l'extérieur de la deuxième unité non-jetable.

Par contre, chacune des trois variantes mentionnées ci-avant permet à un utilisateur de compétences moyennes de réutiliser la première unité, prévue pour être jetée après un seul emploi, s'il en a l'intention. En effet, en bloquant simplement le petit mécanisme de bloquage dans la première variante, ou en isolant à l'aide d'une goutte de colle dans la deuxième variante, ou encore en récupérant la languette conductrice dans la troisième variante et en la fixant à demeure sur les deux bornes externes mentionnées, un utilisateur mal informé ou mal intentionné peut aisément utiliser la première unité plusieurs fois, ceci moyennant un changement de la batterie lorsque cette dernière est déchargée.

Deuxièmement, l'inconvénient mentionné ci-avant est aggravé par le fait qu'un utilisateur a directement accès aux moyens d'enclenchement et d'interruption du fonctionnement du dispositif. Il en résulte que le dispositif est médicalement pas du tout fiable si le médicament n'est pas administré sous contrôle médical, sans que l'utilisateur ait la possibilité d'actionner les moyens d'enclenchement et d'interruption du fonctionnement du dispositif.

Le dispositif décrit dans le document EP 275 213 n'est donc pas approprié pour une pompe portable d'administration d'un médicament ou substance thérapeutique.

L'invention a pour but de remédier aux inconvénients précités.

Elle a donc pour objet une pompe portable d'administration d'une substance thérapeutique liquide comportant les éléments suivants :
- un réservoir de stockage de ladite substance thérapeutique liquide,
- des moyens de pompage permettant de pomper ladite substance thérapeutique stockée dans ledit réservoir de stockage,
- des moyens moteurs pour faire fonctionner lesdits moyens de pompage,
- des moyens de commande agissant sur lesdits moyens moteurs, lesdits élements étant logés dans au moins deux modules initialement séparés et destinés à être assemblés, la pompe portable étant caractérisé en ce qu'elle comprend des moyens de mise en fonctionnement de cette pompe portable agencés de manière que la mise en fonctionnement de cette pompe portable est provoquée uniquement lors de l'assemblage des deux modules, et en ce qu'elle comprend en outre des moyens d'interruption du fonctionnement de ladite pompe portable agencés de telle manière que l'interruption de cette pompe protable est provoquée irréversiblement par le désassemblage des deux modules.

Grâce à ces caractéristiques, tout en restant facilement stérilisable, la pompe selon l'invention présente toutes les garanties de sécurité voulues car,
- dès qu'elle est assemblée, son fonctionnement est enclenché par les seules opérations d'assemblage,
- une fois assemblée, on ne peut la démonter qu'au prix d'une destruction d'un ou de plusieurs éléments essentiels de sa structure, notamment le circuit de commande de la pompe, qu'un utilisateur moyen non averti ne peut absolument pas réparer.

Selon une caractéristique avantageuse, les moyens d'interruption du fonctionnement de la pompe portable sont constitués par des éléments mécaniques portant les moyens de contact électrique et agencés pour se détruire de façon irréversible lorsque les modules sont désassemblés.

Grâce à ces caractéristiques, non seulement la pompe portable est automatiquement mise en route dès l'assemblage des modules, mais un éventuel désassemblage de ceux-ci entraîne la rupture des supports des contacts électriques. Il est donc difficile de faire fonctionner de nouveau la pompe portable puisqu'il faut alors en rassembler les morceaux brisés, puis les reconstituer à l'intérieur de celle-ci.

Selon une autre caractéristique de l'invention, les moyens d'interruption du fonctionnement de la pompe portable sont insérés dans un circuit des moyens de commande.

On s'assure ainsi de façon encore plus fiable qu'une réutilisation de la pompe est impossible puisque l'électronique des moyens de commande est inhibée définitivement dès que les modules sont séparés. Il est en effet pratiquement impossible pour l'utilisateur non averti de fabriquer un nouveau circuit électronique.

De plus, selon encore une autre caractéristique de l'invention, l'un des deux modules assemblables comprend le réservoir de stockage et ce réservoir présente un orifice de remplissage disposé de façon à être inaccessible lorsque ce module est assemblé à l'autre module. Ainsi, on empêche un accès trop facile au réservoir.

En outre, selon une caractéristique supplémentaire de l'invention, le module comprenant le réservoir de stockage est stérilisable avant l'assemblage des deux modules. On peut donc stériliser sélectivement le module contenant le réservoir et ne pas stériliser le (ou les) module(s) comprenant le moteur et le circuit de commande, puisque les techniques de stérilisation sont souvent préjudiciables à l'électronique.

De plus, cette pompe comprend des moyens d'étanchéité capables de rendre la pompe étanche, une fois les deux modules assemblés. Ceci permet à l'utilisateur de conserver en toute sécurité la pompe en permanence sur lui et même, par exemple, de se baigner alors qu'il porte ladite pompe.

Enfin, dans un mode de réalisation préféré, cette pompe portable est une pompe péristaltique comprenant deux modules, un module réservoir comprenant le réservoir de médicament et la (ou les) tubulure(s) reliant le réservoir à la sortie de la pompe et un module moteur comprenant les moyens moteurs, les moyens de commande et un rotor.

De par la conception du mode de réalisation préféré, les deux modules sont stockés séparément et assemblés uniquement au moment de l'utilisation. Ainsi pendant le temps de stockage, les tubulures ne sont pas déformées, ni écrasées par le rotor.

L'invention sera mieux comprise à la lecture de la description suivante, donnée à titre d'exemple illustratif et faite en faisant référence aux dessins joints dans lesquels :
- la figure 1 est un schéma du principe de l'invention,
- la figure 2 est une vue en perspective de la pompe avant l'assemblage de ses deux modules,
- la figure 3 est une vue de dessus de la pompe selon la figure 2, les deux modules étant assemblés et le dessus de la pompe ayant été enlevé,
- la figure 4 est une vue en coupe de la pompe selon la ligne IV-IV de la figure 3,
- la figure 5 est une vue en coupe selon la ligne V-V de la figure 3,
- la figure 6 est une vue en coupe partielle selon la ligne VI-VI de la figure 4,
- les figures 7, 8 et 9 sont des vues de détail, agrandies de la figure 3 dans des zones de la pompe indiquées respectivement par les lettres A, B et C,
- la figure 10 est un schéma du circuit électrique des moyens de commande du moteur de la pompe, et
- la figure 11 illustre une première variante de réalisation des moyens de contact électrique et des moyens d'assemblage représentés en figure 9,
- la figure 12 illustre une seconde variante de réalisation des moyens de contact électrique et des moyens d'assemblage représentés en figure 9.

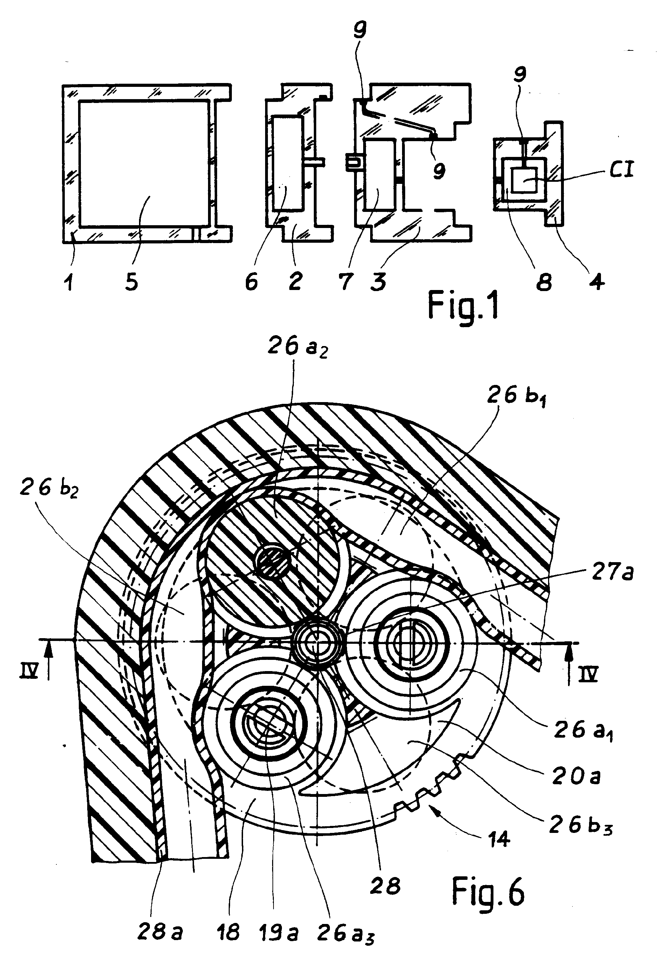

L'invention repose sur un principe dont une variante est représentée schématiquement à la figure 1. Tous les éléments constituant le dispositif de l'invention ont été symbolisés sur cette figure, mais ne reflètent en aucun cas la structure exacte dudit dispositif. La structure particulière est par contre représentée dans les figures suivantes.

Comme illustré à la figure 1, la pompe se compose d'au moins deux modules, ici quatre, référencés 1, 2, 3, 4 assemblables les uns aux autres. Dans le mode de réalisation préféré de l'invention, les modules sont assemblables par glissement d'un des modules dans l'autre et par encliquetage, mais l'on pourrait envisager d'autres moyens, tels que le vissage, l'utilisation d'un système à baïonnette, etc...

Les divers modules abritent respectivement plusieurs éléments, à savoir : un réservoir de stockage 5 de la substance thérapeutique, des moyens de pompage 6 permettant de mener ladite substance thérapeutique depuis le réservoir 5 jusqu'à la sortie de la pompe, des moyens moteurs 7 pour faire fonctionner lesdits moyens de pompage et des moyens de commande 8 agissant sur lesdits moyens moteurs 7. Le réservoir 5, les moyens de pompage 6, les moyens moteurs 7 et les moyens de commande 8 on été répartis et représentés arbitrairement dans les modules 1 à 4, mais il est, bien sûr, évident que le nombre des modules peut varier et que les éléments précités peuvent être répartis différemment dans les modules. Il est cependant avantageux d'isoler dans un ou plusieurs modules les éléments ne supportant pas la stérilisation afin de permettre de faire subir cette opération aux autres modules sans dommage pour les premiers.

En outre, au moins deux de ces modules 1, 2, 3 ou 4 comprennent simultanément des moyens de contact électrique complémentaires 9 provoquant la mise en marche de la pompe lorsque ces modules sont assemblés et des moyens d'interruption irréversible du fonctionnement de la pompe si les moyens de contact 9 sont ensuite désassemblés. De préférence, on disposera les moyens de pompage 6, les moyens moteurs 7 et les moyens de commande 8 dans les modules comprenant ces moyens de contact 9.

Les moyens d'interruption irréversible ne sont que partiellement schématisés sur la figure 1. Selon l'invention, ils peuvent être réalisés sous forme de trois variantes pouvant d'ailleurs être combinées, comme c'est le cas dans le mode de réalisation préféré de l'invention qui va être décrit ci-après.

La première variante peut être mise en oeuvre en prévoyant un circuit intégré CI préprogrammé pour s'arrêter de fonctionner dès que les moyens de contact électrique 9 auxquels il est relié d'une façon particulière, sont séparés, cet arrêt de fonctionnement étant alors rendu irrémédiable par le programme prévu dans le circuit.

La seconde variante peut être mise en oeuvre en prévoyant des supports (non visibles sur la figure 1) des moyens de contact électrique 9, ces supports étant fragiles et agencés de telle façon qu'ils se rompent lors du désassemblage des modules.

La troisième variante peut être réalisée en prévoyant les moyens de contact électrique 9 par exemple sur un premier module et des moyens mécaniques (non visibles sur la figure 1) sur un second module assemblable avec le premier, ces moyens mécaniques étant agencés de façon à rompre lesdits moyens de contact électrique 9 lors du désassemblage desdits modules.

On notera que selon une variante, si le module 4 ne contient aucun élément, il joue le rôle d'interrupteur de mise en marche irréversible de la pompe.

La figure 2 représente une pompe exécutée selon le mode de réalisation préféré de l'invention. Cette pompe ne comporte que deux modules, à savoir un module moteur 11 et un module réservoir 12. Ces deux modules sont susceptibles d'être assemblés. A cet effet, le module réservoir 12 a la forme générale d'un boîtier muni d'une cavité débouchante 13 et le module moteur 11 peut être assimilé à un tiroir qui vient s'engager dans la cavité 13 et qui présente une forme sensiblement complémentaire de celle-ci.

Dans ce mode de réalisation, les moyens de pompage 6 comprennent une pompe péristaltique, mais l'on pourrait envisager d'autres moyens de pompage, sans pour autant sortir du cadre de l'invention. Si les moyens de pompage 6 comprennent une pompe péristaltique, le module moteur 11 comprend un rotor 14 présentant deux étages de galets presseurs, ce rotor étant entraîné par les moyens moteurs 7 (non représenté sur la figure 2).

Le module réservoir 12 comprend le réservoir de liquide 5 relié par un tuyau flexible 15 à un orifice de sortie 15a de la pompe. Cette sortie 15a peut être reliée par exemple à une aiguille hypodermique (non représentée) soit directement, soit par l'intermédiaire d'un tuyau souple (également non représenté), suivant que la pompe est appliquée respectivement sur le thorax ou sur l'abdomen, par exemple.

En outre, le réservoir 5 peut être rempli grâce à un orifice de remplissage 16, réalisé de préférence comme un septum. Pour fixer les idées, la pompe assemblée présente une longueur totale de 55 mm, une largeur totale de 47 mm et et une épaisseur de 14 mm,ses dimensions n'étant bien entendu données qu'à titre d'exemple.

La pompe va maintenant être décrite plus précisément.

Comme illustré à la figure 2, le module moteur 11 est de forme allongée et contient dans sa partie avant plus étroite, le rotor 14 et dans sa partie arrière plus large le moteur 7 et ses moyens de commande 8 (non représentés sur cette figure).

Comme on peut le voir sur la figure 4, le rotor 14 comprend un corps 17 d'un seul tenant et de forme générale cylindrique dont l'axe X-X est également l'axe de rotation. Ce corps 17 définit deux étages 17a et 17b de part et d'autre d'un plan médian radial dans lequel est prévu une couronne dentée 18 destinée à assurer l'entraînement en rotation dudit rotor. Cette couronne déborde de l'enveloppe générale du corps cylindrique qui présente donc son plus grand diamètre à cet endroit.

A chaque étage 17a, 17b du corps 17, il est prévu trois broches 19a, respectivement 19b, d'axe Y-Y parallèle à l'axe X-X. Ces broches 19a, respectivement 19b, sont décalées entre elles de 120° et les broches 19a sont décalées de 60° par rapport aux broches 19b de l'étage inférieur (voir figure 6).

En outre, à chaque étage 17a, 17b, le corps 17 présente trois gorges 20a, respectivement 20b, débouchant à sa surface latérale et ayant sensiblement la forme d'un V à pointe arrondie.

Centrés sur son axe X-X, le corps 17 comporte également deux trous borgnes 21a et 21b dans lesquels sont engagés des pivots respectifs 22a, 22b faisant partie d'un bloc 23 formant la structure portante des moyens moteurs 11.

Chaque broche 19a, 19b présente une fente radiale 24 à son extrémité libre et comporte à cet endroit un rebord annulaire 25 formant un épaulement tourné vers la base de chaque broche.

Le rotor 14 est équipé par étage de trois galets 26a-1, 26a-2, 26a-3, respectivement 26b-1, 26b-2, 26b-3 (voir figure 6) engagés sur les broches respectives 19a et 19b en étant maintenus en place par encliquetage contre l'épaulement 25, tout en pouvant tourner librement sur ces broches. Un léger jeu radial j est prévu pour permettre un certain débattement latéral des galets.

On notera que chaque gorge 20a, 20b est prévue entre deux galets 26a, 26b voisins d'un même étage du rotor 14.

Des ressorts à lame 27a, 27b sont placés dans les espaces délimités entre les galets 26a, 26b et la partie centrale 28 du rotor 14 pour solliciter le galet correspondant vers l'extérieur.

Selon une variante non représentée, les ressorts individuels 27a et 27b peuvent être remplacés par des pièces élastiques uniques à raison d'une par étage pour obtenir le même effet. Une autre variante consiste à les omettre totalement, les galets 26a, 26b étant alors déplacés radialement par l'élasticité inhérante du tuyau 15 lui-même.

En se référant de nouveau à la figure 2, on voit que le tuyau 15 est composé, dans le présent exemple à deux étages de galets, de deux tubulures élémentaires 28a, 28b, une pour chaque étage, et passant autour d'une partie de la périphérie du rotor 14 lorsque les modules 11 et 12 sont assemblés. Ces tubulures 28a, 28b se rejoignent à leurs extrémités correspondantes par des raccords en Y 29 et 30, le raccord 29 étant branché sur le réservoir 5 (côté aspiration de la pompe), tandis que le raccord 30 est en communication avec la sortie 15a (côté refoulement de la pompe).

Les tubulures 28a et 28b sont disposées respectivement dans des gorges 31a, 31b ménagées dans une pièce d'appui 32 en forme de boomerang, adossée à une cloison 33 séparant un compartiment 34 du carter 35 du module réservoir, de la cavité 13 de celui-ci, dans laquelle est inséré le module moteur 11. Le compartiment 34 contient le réservoir 5 proprement dit qui sera décrit ci-après.

Comme on peut le constater en comparant les vues en coupe des figures 4 et 5, le profil des gorges 31a et 31b n'est pas constant, sa concavité étant graduellement plus prononcée en allant de la "pointe" 32a de la pièce d'appui vers les extrémités 32b et 32c de celle-ci (figure 2).

Toutefois, dans une autre variante non représentée, le profil des gorges 31a et 31b est rectiligne et les galets 26a₁, 26a₂, 26a₃, 26b₁, 26b₂, 26b₃ sont cylindriques. Les tubulures 28a et 28b sont donc écrasées entre deux plans parallèles.

Le bloc 23 qui forme la structure portante du module moteur 11 comprend un corps 36 et une plaque de couverture 37 en matière transparente, des parties avancées 38 et 39 en forme de disque venues respectivement de matière avec ce corps et cette plaque de couverture constituant une chape pour permettre le montage en rotation du rotor 14. On rappelle que ce dernier est monté à rotation sur les pivots 22a et 22b venus de matière respectivement avec des disques 38 et 39.

Comme illustré en figure 4, le corps 36 présente une cavité 40 servant de logement aux moyens moteurs 7 et aux moyens de commande 8. Les moyens moteurs comprennent un moteur d'entraînement 41 dont l'arbre de sortie 42 porte un pignon 43 engrénant avec une roue intermédiaire 44 montée à rotation sur un ergot 45 dressé sur le fond de la cavité 40. La roue 44 engrène à son tour avec la couronne dentée 18 du rotor 14.

Les moyens moteurs 7 et les moyens de commande 8 peuvent être construits sur la base d'un mouvement de montre classique dont l'axe de l'aiguille des heures est l'arbre de sortie 42. Son circuit intégré commandant normalement, en coopération avec un quartz, la rotation des aiguilles, est adapté pour être utilisé pour la pompe selon l'invention. Ce mouvement doit donc simplement être modifié quant à son circuit intégré qui doit être programmé spécifiquement pour commander la pompe. Un exemple de mouvement de montre utilisable à cet effet est le calibre 400 fabriqué et vendu par la Société ETA SA Fabriques d'Ebauches, CH-Granges.

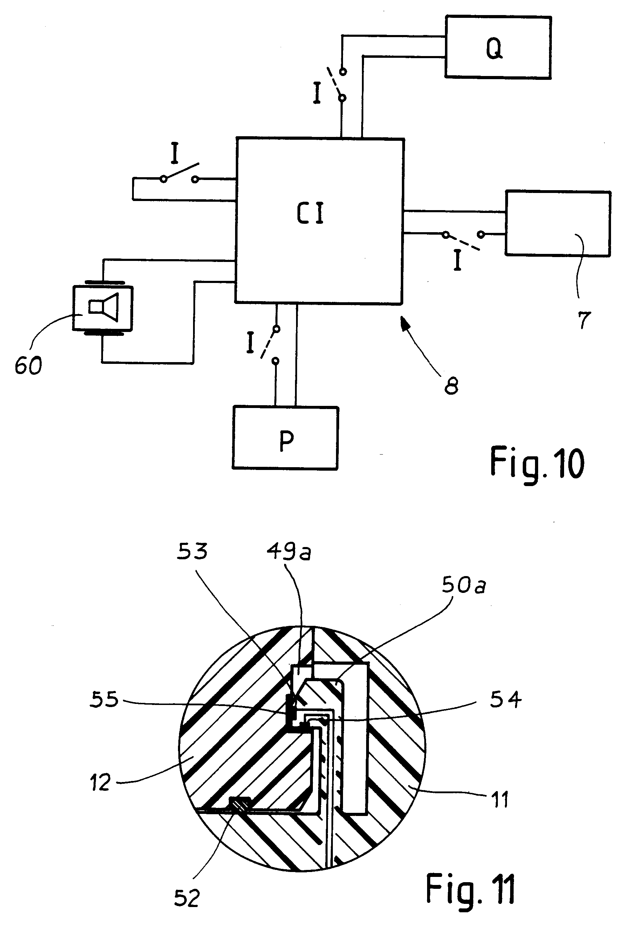

On a représenté sur la figure 10 un schéma électrique d'un tel mouvement de montre dans lequel on reconnaît les moyens de commande 8 comprenant le circuit intégré CI, raccordé d'une part à un quartz Q et d'autre part à une pile d'alimentation P.

Suivant l'invention, les moyens de contact électrique 9 forment un interrupteur I branché sur deux bornes d'entrée du circuit intégré. Dans le cadre du présent mode de réalisation, on admet que lorsque l'interrupteur I est fermé pour la première fois, il déclenche le fonctionnement du circuit intégré CI pour faire démarrer les moyens moteurs 7. Ensuite, lorsque l'interrupteur I est de nouveau ouvert, le circuit intégré interrompt, par sa propre programmation, la commande des moyens moteurs et est désormais incapable de la commander de nouveau. Cette façon de concevoir la programmation du circuit intégré peut facilement être mise en oeuvre par les spécialistes et ne sera donc pas décrite en détail. Selon d'autres variantes schématisées en pointillé, l'interrupteur I pourrait être placé entre le quartz Q et le circuit CI ou entre ce circuit et les moyens moteur 7 ou encore entre le circuit et la pile P.

La figure 9 illustre un mode de réalisation préféré des moyens de contact électrique 9. Le contact mobile de l'interrupteur I est ici matérialisé par une lame élastique 46 fixée sur la paroi latérale du module moteur 11 et coopérant avec un plot de contact 47 fixé en son voisinage et formant le contact fixe de l'interrupteur I. On conçoit facilement que l'interrupteur I ainsi conçu est fermé dès que le module moteur 11 est inséré dans la cavité 13, la paroi latérale de celle-ci faisant alors office de poussoir de la lame élastique 46.

La figure 9 montre également des moyens d'assemblage 48 des modules 11 et 12 qui sont prévus au-dessus des moyens de contact électriques 9, légèrement en retrait par rapport à ceux-ci dans le sens de l' insertion SI du module 11.

Ces moyens d'assemblage comprennent d'une part deux creusures 49 pratiquées respectivement dans les parois latérales de la cavité 13 et, d'autre part, deux crochets élastiques 50 venus de matière avec le corps du module moteur 11, chaque crochet venant s'accrocher dans l'une des creusures 49 après insertion complète du module 11 dans le module 12.

On notera que si on tente de sortir le module 11 du module 12 après un premier assemblage, les crochets 50 se casseront, ce qui empêche tout nouvel assemblage solide des deux modules.

Comme représenté clairement sur la figure 2, le corps 36 est pourvu d'une tête de préhension 51 qui en est venu de formage et qui, tout en facilitant la manipulation du module moteur 11, obstrue complètement la cavité débouchante 13. Ceci a pour conséquences que cette cavité 13 devient inaccessible dès l'assemblage des deux modules, que l'orifice de remplissage 16 est définitivement caché et que l'on ne peut plus atteindre ni les moyens de contact électrique 9, ni les moyens d'assemblage 48.

Par ailleurs, un joint 52 entoure l'embouchure de la cavité 13, contre lequel vient s'appuyer la face intérieure de la tête de préhension 51, l'élasticité de ce joint assurant, de préférence, l'accrochage à force des crochets 50 dans les creusures 49 dans le sens opposé à la flèche SI (figure 9).

La figure 11 illustre un second mode de réalisation des moyens de contact électrique et des moyens d'interruption irréversible du fonctionnement de la pompe.

Le module moteur 11 présente de chaque côté un crochet 50a de forme identique à celui représenté sur la figure 9. Le module réservoir 12 présente également une creusure 49a d'accrochage du crochet 50a.

L'un des crochets 50a porte à son extrémité deux contacts électriques distincts 53, 54 reliés aux bornes du circuit intégré CI conformément au schéma de la figure 10, par des connexions non représentées. Ce crochet 50a constitue donc un élément mécanique support des moyens de contact électrique. Le fond de la creusure 49a présente une lame métallique continue 55 et la forme de cette creusure est conçue pour que les contacts 53 et 54 puissent être reliés ensemble par la lame 55 lorsque les deux modules sont assemblés, ce qui a pour effet de mettre la pompe en marche.

Comme vu précédemment pour la figure 9, la séparation du module moteur 11 et du module réservoir 12, entraîne ensuite la rupture du crochet 50a et rend l'interruption du fonctionnement de la pompe irréversible, puisqu'il sera alors très difficile de recoller le crochet 50a et de rétablir les liaisons électriques jusqu'au circuit CI.

Ce dispositif peut être un deuxième moyen d'interruption irréversible du fonctionnement de la pompe, qui n'est pas incompatible avec la programmation du circuit intégré vue précédemmant. Cependant, dans ce mode de réalisation, on peut aussi se dispenser éventuellement de programmer le circuit CI de manière qu'il se bloque automatiquement après rupture de la liaison assurée par les contacts 53 et 54 et la lame 55.

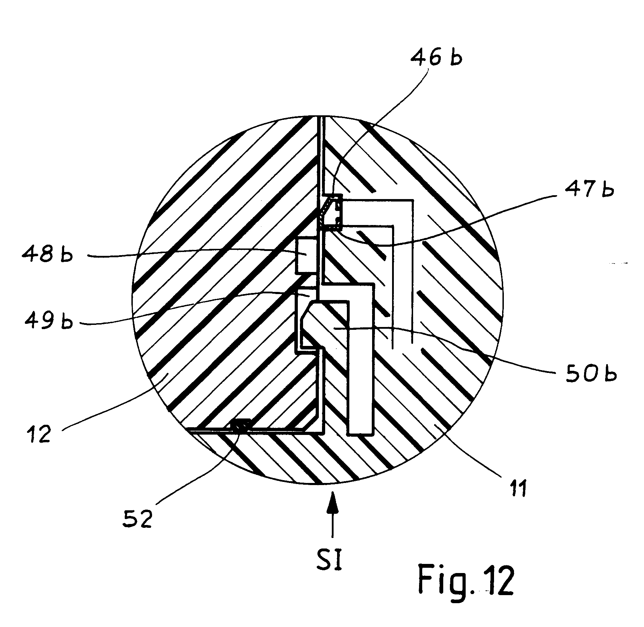

La figure 12 illustre un troisième mode de réalisation des moyens de contact électrique et des moyens d'interruption irréversible du fonctionnement de la pompe.

De façon similaire à ce qui a été décrit pour la figure 11, le module moteur 11 présente de chaque côté un crochet 50b et le module réservoir 12 présente également de chaque côté de la cavité 13, une creusure 49b d'accrochage dudit crochet 50b. Ce crochet 50b et cette creusure 49b ont une forme identique à ceux représentés en figure 9.

Le contact mobile de l'interrupteur I est ici matérialisé par une lame élastique conductrice 46b fixée dans la paroi latérale du module moteur 11 et coopérant avec un plot de contact 47b fixé en son voisinage et formant le contact fixe de l'interrupteur I. Cette lame 46b et ce plot 47b sont reliés par des connexions aux bornes du circuit intégré CI (non représenté) conformément au schéma de la figure 10. On comprend aisément que l'interrupteur I ainsi conçu est fermé dès que la module moteur 11 est introduit dans la cavité 13 du module 12, puisque la paroi latérale de cette cavité fait office de poussoir de la lame élastique 46b. Ainsi, dès l'assemblage des deux modules 11, 12, la mise en marche est automatique. Par ailleurs, le module 12 présente une seconde creusure 48b prévue légèrement en retrait par rapport à la lame 46b dans le sens d'insertion SI du module 11.

Si après un premier assemblage des deux modules, on tente de sortir le module 11 du module 12 (sens opposé à SI), non seulement les crochets élastiques 50b retenus par les creusures 49b se briseront mais la lame élastique 46b de par sa forme et son élasticité aura tendance à s'écarter vers l'extérieur et à se rompre également, en étant bloquée dans la seconde creusure 48b. On interrompt ainsi de façon irréversible le fonctionnement de la pompe, puisqu'il est alors très difficile voire impossible de recoller la lame 46b et de rétablir les liaisons électriques jusqu'au circuit CI.

Enfin, on notera que ce troisième moyen d'interruption irréversible du fonctionnement de la pompe, n'est pas incompatible avec la programmation du circuit intégré, vue précédemment.

Dans les trois modes de réalisation des moyens de contact électrique et des moyens d'interruption irréversible du fonctionnement de la pompe qui viennent d'être décrits, il est évident que les éléments qui sont prévus sur le module 11 pourraient être placés sur le module 12 et inversément.

Une description plus précise du module réservoir 12 va maintenant être faite. Comme on peut le voir sur les figures 2, 3, 7 et 8, le module réservoir 12 délimite un logement 56 dans lequel est placé le réservoir 5 constitué par une vessie en matière plastique souple, par exemple en PVC, recouverte d'un revêtement étanche (non représenté).

Le volume préféré de la vessie 5 est de 10 cm³, correspondant à 8 cm³ de liquide à injecter (à raison d'un 1 cm³/jour pendant 8 jours par exemple) et à 2 cm³ de réserve. Ce volume n'est donné qu'à titre indicatif.

Le logement 56 se prolonge à l'une de ses extrémités par un canal 57 (figure 8) permettant de guider et de soutenir une portion de tuyau 58. Ce tuyau 58 relie la vessie 5 au raccord en Y d'entrée 29 (figure 2).

De façon similaire, un autre canal 59 guide un tuyau (non représenté) qui relie le raccord en Y de sortie 30 à la sortie 15a. Ces canaux 57 et 59 s'interrompent au niveau du fond de la cavité débouchante 13 pour que les deux tubulures 28a et 28b puissent être écrasées directement par le rotor 14 dans les gorges d'appui 31a et 31b précédemment décrites.

En outre, la vessie 5 présente du côté opposé à celui du tuyau 58, le septum 16 débouchant à côté de la cavité 13.

La pompe est munie d'une ou plusieurs alarmes sonores et visuelles pouvant indiquer son éventuel mauvais fonctionnement. Ces alarmes vont maintenant être décrites.

Comme illustré aux figures 4 et 10, l'alarme sonore comprend un vibreur 60 disposé de préférence dans un logement 61 prévu à cet effet dans la plaque de couverture 37 du module moteur 11, au-dessus des moyens moteurs 7. Le vibreur 60 pourrait également être disposé dans le module réservoir 12 si cela permet à l'utilisateur d'entendre plus facilement l'alarme sonore. Cette alarme 60 est reliée aux bornes du circuit intégré CI qui la commande dès qu'il détecte le début du fonctionnement de la pompe, la fin du fonctionnement, une panne du moteur ou une obstruction de l'une des tubulures (28a, 28b). On peut également équiper le circuit CI d'un circuit de détection de la fin de vie de la pile P et faire sonner l'alarme 60 lorsque ce circuit est activé.

On prévoit également une alarme visuelle 62 pouvant indiquer un éventuel mauvais fonctionnement ou arrêt du rotor 14 ou des moyens moteurs 7 (figures 2 et 4). Cette alarme comprend un disque 63 dont la face supérieure présente sur la périphérie un point de couleur 64 ce disque étant fixé sur l'arbre des minutes 64a du mouvement de montre, cet arbre étant coaxial avec l'arbre 42. Ce dernier traverse un trou 65 prévu dans le corps 36. Ainsi, toute variation de rotation des moyens moteurs 7 ou du rotor 14 se traduit par une variation correspondante de la vitesse de rotation du disque 63, voire son arrêt pur et simple.

Afin que la périphérie du disque 63 puisse être vue de l'extérieure, la plaque de couverture 37 du module moteur 11 et le module réservoir 12 lui-même sont réalisés en matière plastique transparente, le vibreur 60 recouvrant la majeure partie du disque 64.

Toutefois, si le vibreur 60 est placé dans le module 12, la totalité du disque 63 sera visible.

Enfin, pour éviter que le module moteur 11 ne soit introduit à l'envers dans le module réservoir 12, on prévoit par exemple sur l'une des faces du module moteur 11 des saillies longitudinales 66 coopérant avec des nervures longitudinales 67 prévues sur la face en regard de la cavité débouchante 13.

Le fonctionnement de la pompe que l'on vient de décrire est le suivant.

Si la pompe est utilisée en milieu hospitalier, le personnel soignant peut disposer d'une réserve de modules 11 non stérilisés et de modules 12 stérilisés, emballés séparément. L'infirmière devant équiper un malade d'une pompe selon l'invention, remplit d'abord d'un médicament approprié la vessie 5 à l'aide d'un seringue à travers le septum 16 jusqu'à ce qu'elle aperçoive le médicament à la sortie 15a, ce qui purge la pompe de l'air qui y était contenu. Cette purge est facilitée par la forme en croissant de la vessie 5.

Puis elle engage le module moteur 11 dans la cavité 13, ce qui a pour conséquence,
- la mise en marche de la pompe par l'intermédiaire des moyens de contact électrique 9,
- le blocage du module 11 dans le module 12 à l'aide des crochets 50 qui s'accrochent dans les creusures 49.

Bien entendu, immédiatement après, elle place la pompe sur le patient à l'aide d'une aiguille hypodermique.

En observant la figure 6, on voit que dès la mise en marche, le rotor 14 commence à tourner, les galets 26a et 26b écrasant tour-à-tour les tubulures 28a et 28b respectives selon un mouvement progressif qui est dû au profil évolutif des gorges 31a, 31b de la pièce d'appui 32. Les tubulures, dans les zones situées entre les galets 26a, 26b peuvent s'effacer dans les gorges 20a, 20b du rotor 14. De plus l'écart de 60° entre les galets 26a et les galets 26b permet de régulariser le débit de sortie.

A titre d'exemple, si le quartz Q utilisé dans les moyens de commande 8 est accordé sur une fréquence de 32768 Hz, on peut obtenir après six divisions par deux et une division par cinq, une fréquence de commande du moteur d'entraînement (qui est du type pas à pas) de 102,4 Hz. Le moteur 41 étant du type monophasé bipolaire, son rotor fait deux pas par tour, ce qui, avec une réduction appropriée du rouage formé par la roue 44 et la couronne 18 donne au rotor 14 une vitesse d'un tour toutes les 36 minutes environ. Le débit moyen régulier de la pompe peut alors être de 1 cm³ en 24 heures. En changeant le diamètre interne des tubulures 28a, 28b (mais en conservant la même épaisseur de tube), on pourrait également obtenir des débits d'environ 3cm² en 24 heures.

## Revendications

1. Pompe portable d'administration d'une substance thérapeutique liquide comportant les éléments suivants :
- un réservoir de stockage (5) de ladite substance thérapeutique liquide,
- des moyens de pompage (6) permettant de pomper ladite substance thérapeutique liquide stockée dans ledit réservoir de stockage (5) jusqu'à la sortie (15a) de la pompe,
- des moyens moteurs (7) pour faire fonctionner lesdits moyens de pompage,
- des moyens de commande (8) agissant sur lesdits moyens moteurs,
lesdits élements étant logés dans au moins deux modules (11,12) initialement séparés et destinés à être assemblés, la pompe portable étant caractérisée en ce qu'elle comprend des moyens de mise en fonctionnement (46,47;46b,47b;53,54,55) de cette pompe portable agencés de manière que la mise en fonctionnement de cette pompe portable est réalisée par la seule opération d'assemblage des deux modules, et en ce qu'elle comprend en outre des moyens d'interruption (49a,50a;48b,46b) du fonctionnement de ladite pompe portable agencés de telle manière que l'interruption de cette pompe portable est provoquée irréversiblement par le premier désassemblage des deux modules (11,12).

2. Pompe portable selon la revendication 1, caractérisée en ce que lesdits moyens de mise en fonctionnement sont formés par des premiers et des deuxièmes moyens de contact électrique (46,47;46b, 47b;53,54,55) complémentaires, les deux modules (11,12) étant agencés de manière que ces premiers et deuxièmes moyens de contact électrique sont mis en contact électrique lors de l'assemblage des deux modules.

3. Pompe portable selon la revendication 1 ou 2, caractérisée en ce que lesdits moyens d'interruption du fonctionnement sont insérés dans un circuit (CI) des moyens de commande (8).

4. Pompe portable selon la revendication 1 ou 2, caractérisée en ce que lesdits moyens d'interruption du fonctionnement (49a,50a) sont formés par des éléments mécaniques (50a), portant au moins lesdits premiers moyens de contact électrique (53, 54), qui se détruisent de façon irréversible lorsque les deux modules (11, 12) sont désassemblés.

5. Pompe portable selon la revendication 1 ou 2, caractérisée en ce que lesdits moyens d'interruption du fonctionnement comprennent des éléments mécaniques (48b), prévus dans l'un des deux modules (11, 12), qui détruisent de façon irréversible au moins lesdits deuxièmes moyens de contact électrique (47b), prévus dans l'autre module, lorsque lesdits modules sont désassemblés.

6. Pompe portable selon l'une quelconque des revendications précédentes, caractérisée en ce que l'un (12) des deux modules (11,12) assemblables comprend ledit réservoir de stockage (5) et en ce que ce réservoir de stockage comprend un orifice de remplissage (16) disposé de façon à être inaccessible lorsque ledit module est assemblé à l'autre module.

7. Pompe portable selon l'une quelconque des revendications précédentes, caractérisée en ce que les moyens de commande (8) agissant sur les moyens moteurs (7) comprennent un circuit intégré (CI) raccordé à un quartz (Q), à une pile d'alimentation (P) et à un interrupteur (I) formé par les moyens de mise en fonctionnement de la pompe portable (46, 47; 46b, 47b; 53, 54, 55).

8. Pompe portable selon les revendications 3 et 7, caractérisée en ce que le circuit intégré (CI) est conçu pour cesser de faire fonctionner les moyens moteurs (7) de façon irréversible dès l'ouverture de l'interrupteur (I).

9. Pompe portable selon l'une quelconque des revendications précédentes, caractérisée en ce que des moyens d'assemblage des deux modules (11, 12) sont constitués par au moins un crochet élastique (50, 50b) prévu sur l'un des deux modules (11, 12) et par au moins une creusure (49, 49b) prévue dans l'autre module, ce crochet (50, 50b) venant s'accrocher dans la creusure (49, 49b) après insertion complète d'un des deux modules dans l'autre.

10. Pompe portable selon la revendication 1, caractérisée en ce que les moyens de pompage (6) sont constitués par une pompe péristaltique et comprennent un rotor (14) présentant au moins un étage (17a, 17b) d'au moins un galet (26a, 26b) et une couronne dentée (18) coopérant avec les moyens moteurs (7), ces galets compressant localement au moins une tubulure (28a, 28b) reliant le réservoir (5) à une sortie (15a) de la pompe, cette compression étant effectuée contre au moins une gorge (31a, 31b) d'une pièce d'appui (32).

11. Pompe portable selon la revendication 10, caractérisée en ce qu'elle comprend un module moteur (11) comprenant le rotor (14), les moyens moteurs (7), les moyens de commande (8) et un module réservoir (12) comprenant le réservoir de stockage (5) muni de son orifice de remplissage (16), d'au moins une tubulure (28a, 28b) et la pièce d'appui (32).

12. Pompe portable selon la revendication 11, caractérisée en ce qu'elle comprend deux tubulures flexibles (28a, 28b) connectées en parallèle à chacune de leurs extrémités par un raccord en Y (29) pour former une entrée commune au niveau du réservoir (5) et par un raccord en Y (30) pour aboutir à une sortie commune (15a) et en ce que le rotor (14) comprend deux étages (17a, 17b) de galets presseurs, les galets (26a1, 26a2, 26a3) du premier étage étant décalés angulairement par rapport aux galets ( 26b1, 26b2, 26b3) du second étage, pour régulariser le débit de sortie.

13. Pompe portable selon la revendication 6, caractérisée en ce que le module (12) contenant le réservoir (5) est stérilisable avant l'assemblage avec l'autre module.

14. Pompe portable selon l'une quelconque des revendications précédentes, caractérisée en ce que le réservoir de stockage (5) est constitué par une vessie en matière plastique souple étanche.

15. Pompe portable selon la revendication 7 caractérisée en ce qu'elle comprend au moins une alarme sonore du mauvais fonctionnement des moyens moteurs (7), cette alarme comprenant un vibreur (60) connecté au circuit (CI).

16. Pompe portable selon la revendication 15, caractérisée en ce que le circuit intégré (CI) comprend un circuit de détection de fin de vie de la pile (P), faisant fonctionner le vibreur (60) lorsqu'il est activé.

17. Pompe portable selon l'une quelconque des revendications précédentes, caractérisée en ce qu'elle comprend au moins une alarme visuelle (62) du mauvais fonctionnement des moyens moteurs (7), cette alarme (62) comprenant un disque (63) fixé sur un arbre de sortie des moyens moteurs (7) et entraîné en rotation par ceux-ci.

18. Pompe portable selon la revendication 1, caractérisée en ce qu'elle comprend des moyens d'étanchéité (52), capables de rendre la pompe étanche une fois les deux modules (11, 12) assemblés.

## Patentansprüche

1. Tragbare Pumpe zum Verabreichen einer flüssigen, therapeutischen Substanz, umfassend die folgenden Elemente:
- ein Speicherreservoir (5) der flüssigen, therapeutischen Substanz,
- Pumpenmittel (6), die es ermöglichen, die flüssige, therapeutische Substanz, die in dem Reservoir (5) aufbewahrt wird, bis zum Auslaß (15a) der Pumpe zu pumpen,
- Motormittel (7) zum Betreiben der Pumpenmittel,
- Steuermittel (8), die auf die Motormittel einwirken, welche Elemente in mindestens zwei Modulen (11, 12) untergebracht sind, die anfänglich getrennt sind und zusammengefügt werden können, welche tragbare Pumpe dadurch gekennzeichnet ist, daß sie Inbetriebnahmemittel (46, 47; 46b, 47b; 53, 54, 55) dieser tragbaren Pumpe umfaßt, welche so ausgebildet sind, daß die Inbetriebnahme dieser tragbaren Pumpe ausschließlich durch das Zusammenfügen der beiden Module realisiert wird, und daß sie ferner Unterbrechermittel (49a, 50a; 48b, 46b) für die Funktion der tragbaren Pumpe umfaßt, die derart ausgebildet sind, daß die Unterbrechung der tragbaren Pumpe irreversibel durch erstmaliges Demontieren der beiden Module (11, 12) hervorgerufen wird.

2. Tragbare Pumpe nach Anspruch 1, dadurch gekennzeichnet, daß die Inbetriebnahmemittel von ersten und zweiten elektrischen Kontaktmitteln (46, 47; 46b, 47b; 53, 54, 55), die zueinander komplementär sind, gebildet werden, wobei die beiden Module (11, 12) derart ausgebildet sind, daß diese ersten und zweiten elektrischen Kontaktmittel in elektrischen Kontakt gebracht werden beim Zusammenfügen der beiden Module.

3. Tragbare Pumpe nach Anspruch 1 oder 2, dadurch gekennzeichnet, daß die Funktionsunterbrechungsmittel in einen Schaltkreis (CI) der Steuermittel (8) eingefügt sind.

4. Tragbare Pumpe nach Anspruch 1 oder 2, dadurch gekennzeichnet, daß die Funktionsunterbrechungsmittel (49a, 50a) von mechanischen Elementen (50a) gebildet werden, die mindestens ein erstes elektrisches Kontaktmittel (53, 54) tragen, welche sich in irreversibler Weise zerstören, wenn die beiden Module (11, 12) demontiert werden.

5. Tragbare Pumpe nach Anspruch 1 oder 2, dadurch gekennzeichnet, daß die Funktionsunterbrechungsmittel mechanische Elemente (48b) umfassen, vorgesehen in einem der beiden Module (11, 12), welche in irreversibler Weise mindestens die genannten zweiten elektrischen Kontaktelemente (47b) zerstören, die in dem anderen Modul vorgesehen sind, wenn die Module demontiert werden.

6. Tragbare Pumpe nach einem der vorangehenden Ansprüche, dadurch gekennzeichnet, daß einer (12) der beiden zusammenfügbaren Module (11, 12) das Speicherreservoir (5) umfaßt und daß das Speicherreservoir eine Nachfüllöffnung (16) umfaßt, die derart angeordnet ist, daß sie unzugänglich ist, wenn der Modul mit dem anderen Modul zusammengefügt ist.

7. Tragbare Pumpe nach einem der vorangehenden Ansprüche, dadurch gekennzeichnet, daß die auf die Motormittel (7) einwirkenden Steuermittel (8) einen integrierten Schaltkreis (CI) umfassen, der an einen Quarz (Q), an eine Speisebatterie (P) und einen Unterbrecher (I) angeschlossen ist, gebildet von den Inbetriebnahmemitteln der tragbaren Pumpe (46, 47; 46b, 47b; 53, 54, 55).

8. Tragbare Pumpe nach Ansprüchen 3 und 7, dadurch gekennzeichnet, daß der integrierte Schaltkreis (CI) ausgebildet ist, um den Betrieb der Motormittel (7) in irreversibler Weise beim öffnen des Unterbrechers (I) zu beenden.

9. Tragbare Pumpe nach einem der vorangehenden Ansprüche, dadurch gekennzeichnet, daß die Fügemittel der beiden Module (11,12) von mindestens einem elastischen Haken (50, 50b) gebildet werden, der auf einem der beiden Module (11, 12) vorgesehen ist, und von mindestens einer Falle (49, 49b), die an dem anderen Modul ausgebildet ist, wobei der Haken (50, 50b) sich in der Falle (49, 49b) verankert nach vollständiger Einfügung des einen Moduls in den anderen.

10. Tragbare Pumpe nach Anspruch 1, dadurch gekennzeichnet, daß die Pumpenmittel (6) von einer peristaltischen Pumpe gebildet werden und einen Rotor (14) umfassen, der mindestens eine Etage (17a, 17b) mindestens eines Rollkörpers (26a, 26b) und eine verzahnte Krone (18) umfaßt, die mit den Motormitteln (7) zusammenwirkt, welche Rollkörper lokal mindestens einen Schlauch (28a, 28b) komprimieren, der das Reservoir (5) mit einem Auslaß (15a) der Pumpe verbindet, welche Kompression gegen mindestens eine Nut (31a, 31b) eines Stützteils (32) ausgeführt wird.

11. Tragbare Pumpe nach Anspruch 10, dadurch gekennzeichnet, daß sie einen Motormodul (11), welcher den Rotor (14), die Motormittel (7), die Steuermittel (8) enthält, und einen Reservoirmodul (12) umfaßt, welcher das Speicherreservoir (5), versehen mit seiner Nachfüllöffnung (16), mindestens einen Schlauch (28a, 28b) und das Stützteil (32) umfaßt.

12. Tragbare Pumpe nach Anspruch 11, dadurch gekennzeichnet, daß sie zwei flexible Schläuche (28a, 28b) umfaßt, die an jedem ihrer Enden über eine Y-Verbindung (29) parallelgeschaltet sind zum Bilden eines gemeinsamen Einlasses in Höhe des Reservoirs (5) und mittels eines Y-Verbinders (30) zum Verbinden mit einem gemeinsamen Auslaß (15a), und daß der Rotor (14) zwei Etagen (17a, 17b) von pressenden Rollkörpern umfaßt, wobei die Rollkörper (26a1, 26a2, 26a3) der ersten Etage winkelversetzt sind gegenüber den Rollkörpern (26b1, 26b2, 26b3) der zweiten Etage zwecks Vergleichmäßigung des Durchsatzes am Auslaß.

13. Tragbare Pumpe nach Anspruch 6, dadurch gekennzeichnet, daß der das Reservoir (5) enthaltene Modul (12) vor dem Zusammenfügen mit dem anderen Modul sterilisierbar ist.

14. Tragbare Pumpe nach einem der vorangehenden Ansprüche, dadurch gekennzeichnet, daß das Speicherreservoir (5) von einer Blase aus abgedichtetem, weichem Kunststoffmaterial gebildet wird.

15. Tragbare Pumpe nach Anspruch 7, dadurch gekennzeichnet, daß sie mindestens einen Audioalarm der Fehlfunktion der Motormittel (7) umfaßt, welcher Alarm einen mit dem Schaltkreis (CI) verbundenen Vibrator (60) umfaßt.

16. Tragbare Pumpe nach Anspruch 15, dadurch gekennzeichnet, daß der integrierte Schaltkreis (CI) eine Schaltung zum Erkennen des Endes der Lebensdauer der Batterie (P) umfaßt, welche den Vibrator (60) bei Aktivierung arbeiten läßt.

17. Tragbare Pumpe nach einem der vorangehenden Ansprüche, dadurch gekennzeichnet, daß sie mindestens einen Videoalarm (62) umfaßt hinsichtlich der Fehlfunktion der Motormittel (7), welcher Alarm (62) eine Scheibe (63) umfaßt, die an einer Ausgangswelle der Motormittel (7) befestigt ist und von diesen zur Drehung angetrieben wird.

18. Tragbare Pumpe nach Anspruch 1, dadurch gekennzeichnet, daß sie Abdichtmittel (52) umfaßt, die die Pumpe abdichten, wenn einmal die beiden Module (11, 12) gefügt sind.

## Claims

1. Portable pump for the administration of a therapeutic liquid substance comprising the following features :
- a supply reservoir (5) for the therapeutic liquid substance,
- pumping means (6) permitting provision of said therapeutic liquid substance from the supply reservoir (5) up to the pump exit (15a),
- motor means (7) for setting into operation said pump means,
- control means (8) acting on said motor means,
said features being disposed in at least two modules initially separated and intended to be assembled, the portable pump being characterized in that it comprises means for setting into operation this portable pump and arranged in a manner such that the setting into operation of this portable pump is obtained solely by assembling the two modules, and in that it further comprises disconnection means for disconnecting the operation of said portable pump and arranged in a manner such that the disconnection of this portable pump is caused by the first disassembling of the two modules in a permanent manner.

2. Portable pump according to claim 1, characterized in that said means for setting into operation are formed by first and second complementary electric contact means, the two modules being arranged in such a manner that these first and second electric contact means are connected electrically when assembling the two modules.

3. Portable pump according to claim 1 or 2, characterized in that said permanent disconnection means are incorporated into a circuit (CI) of the control means (8).

4. Portable pump according to claim 1 or 2, characterized in that said permanent disconnection means (49a, 50a) are made out of mechanical elements (50a) carrying at least said first electrical contact means (53, 54) which are destroyed in a permanent manner when the two modules (11, 12) are disassembled.

5. Portable pump according to claim 1 or 2, characterized in that said permanent disconnection means comprise mechanical elements (48b) incorporated into one of the modules (11, 12) which destroy at least said second electrical contact means (47b) provided in the other module in a permanent manner when said modules are disassembled.

6. Portable pump according to any one of the preceding claims, characterized in that one of the two assembled modules (11, 12) comprises the supply reservoir (5) and in that such supply reservoir has a filling orifice (16) disposed in a manner such that it is rendered inaccessible when said module is assembled with the other module.

7. Portable pump according to any one of the preceding claims, characterized in that the control means (8) acting on the motor means (7) comprise an integrated circuit (CI) connected to a quartz (Q), to a battery (P) and to a switch (I) formed by the means of setting into operation the portable pump (46, 47; 46b, 47b; 53, 54, 55).

8. Portable pump according to claims 3 and 7, characterized in that the integrated circuit (CI) is so constructed that it stops operation of the motor means (7) in a permanent manner when the switch (I) is opened.

9. Portable pump according to any one of the preceding claims, characterized in that the assembly means of the two modules (11, 12) are formed by at least one elastic hook (50, 50b) provided on one of the two modules (11, 12) and by at least one hollow (49, 49b) provided on the other module, such hook (50, 50b) being adapted to hook into the hollow (49, 49b) after complete insertion of one of the two modules into the other.

10. Portable pump according to claim 1, characterized in that the pump means (6) consist of a peristaltic pump and comprise a rotor (14) having at least one level (17a, 17b) and at least one roller (26a, 26b) and a toothed crown (18) cooperating with the motor means (7), said roller pressing at least locally against the tube (28a, 28b) coming from the reservoir (5) to an exit (15a) of the pump, this compression being effected against at least one groove (31a, 31b) of a bearing member (32).

11. Portable pump according to claim 10, characterized in that it contains a motor module (11) containing the rotor (14), the motor means (7), the control means (8) and a supply module (12) having the supply reservoir (5) equipped with its filling orifice (16), at least one tube (28a, 28b) and the bearing member (32).

12. Portable pump according to claim 11, characterized in that it comprises two flexible tubes (28a, 28b) connected in parallel at each of their extremities with a Y-connector (29), thereby to form a joint entrance to the reservoir (5) and by a Y-connector (30) leading to a common exit (15a) and in that the rotor has two levels(17a, 17b) of pressure rollers, the rollers (26a1, 26a2, 26a3) of the first stage being displaced in an angular manner with respect to the rollers (26b1, 26b2, 26b3) of the second stage, in order to provide a regular supply of liquid from the exit.

13. Portable pump according to claim 6, characterized in that the module (12) containing the supply reservoir (5) is sterilizable before assembly with the other module.

14. Portable pump according to any one of the preceding claims, characterized in that the supply reservoir (5) is made of a bag of supple sealed plastic material.

15. Portable pump according to claim 7, characterized in that it comprises at least one audio alarm for the malfunctioning of the motor means (7), this alarm comprising a vibrator (60) connected to the circuit (CI).

16. Portable pump according to claim 15, characterized in that the integrated circuit (CI) comprises a circuit for the detection of the end of the battery (P) life thereby activating a vibrator (60).

17. Portable pump according to any one of the preceding claims, characterized in that it comprises at least one visual alarm (62) for the malfunctioning of the motor means (7), this alarm (62) comprising a disk (63) fixed to the arm at the exit of the motor means (7) and being rotatably carried by the latter.

18. Portable pump according to claim 1, characterized in that it comprises sealing means (52) for sealing the pump means when the two modules (11, 12) are assembled.
